# EUROPEAN PATENT APPLICATION

(11) **EP 1 978 030 A1**
(43) Date of publication of application: **08.10.2008**
(21) Application number: 07706959.9
(22) Date of filing: 18.01.2007
(51) Int. Cl.: C07K 14/505, C07K 1/12

(54) **METHOD OF REMOVING SIALIC ACID AND PROCESS FOR PRODUCING ASIALOERYTHROPOIETIN**

(30) Priority: 18.01.2006 JP 2006010367
(71) Applicant: CHUGAI SEIYAKU KABUSHIKI KAISHA, Tokyo, 115-8543 (JP)
(72) Inventor: HIGUCHI, Masato, Numazu-shi, Shizuoka 410-0012 (JP)
(74) Representative: Vossius & Partner
(86) International application number: PCT/JP2007/050655
(87) International publication number: WO 2007/083683

(57) **Abstract**

The object is to provide a method for removing a sialic acid that can be carried out simply at a low cost and a method for producing asialoerythropoietin using the same. The method for removing a sialic acid according to the present invention includes an acidifying and heating process of acidifying and heating a solution containing erythropoietin so as to remove a sialic acid bonded to an erythropoietin molecule. Further, the method for producing asialoerythropoietin according to the present invention includes a sialic acid removing process of removing a sialic acid bonded to an erythropoietin molecule by the method for removing a sialic acid according to the present invention.

## Description

### Technical Field

The present invention relates to a method for removing a sialic acid and a method for producing asialoerythropoietin.

### Background Art

Erythropoietin (in the following, also referred to as EPO) is an acid glycoprotein hormone that has a function of promoting the differentiation and proliferation of erythroid precursor cells and is produced mainly from a kidney. Erythrocytes are present most abundantly in blood, and destroyed in a spleen, etc. after functioning for a certain period. For example, the average lifetime of human erythrocytes is about 120 days. On the other hand, erythrocytes are supplied constantly from a bone marrow, and the total number of peripheral erythrocytes always is kept constant under a normal condition. The erythropoietin plays a central role in maintaining such erythrocytic homeostasis in an organism. Clinically, the erythropoietin is utilized in a therapy for anemia and management before and after an operation.

In recent years, studies have been conducted on the replacement of erythropoietin having a natural sugar chain by asialoerythropoietin, which is obtained by removing a sialic acid bonded to erythropoietin, for a clinical use (for example, see Patent document 1 (corresponding to WO 2002/053580)). This asialoerythropoietin is produced usually by treating erythropoietin with an enzyme such as sialidase or can be produced by using a sialyltransferase deficient cell as a host cell (for example, see Patent document 1). The asialoerythropoietin is metabolized promptly via asialoglycoprotein receptors present in a liver. Thus, even when the asialoerythropoietin leaks out from an administration site into blood, no erythropoiesis occurs, so that the asialoerythropoietin can be applied clinically also in a disease in which polycythemia and blood pressure elevation exacerbate the symptoms.
Patent document 1: JP 2005-502584 A

### Disclosure of Invention

### Problem to be Solved by the Invention

However, the above-described conventional method for removing a sialic acid using an enzyme has a problem of the process being complicated, resulting in a high cost. Similarly, the method using a specific host cell has a problem of increased complication of the process and a problem of increased cost.

Then, the object of the present invention is to provide a method for removing a sialic acid that can be carried out simply at a low cost and a method for producing asialoerythropoietin using the same.

### Means for Solving Problem

In order to achieve the above-mentioned object, a method for removing a sialic acid according to the present invention is a method for removing a sialic acid bonded to erythropoietin, including an acidifying and heating process of acidifying and heating a solution containing the erythropoietin. Alternatively, it is a method for removing a sialic acid further including a neutralizing and cooling process of neutralizing and cooling the solution containing the erythropoietin after the acidifying and heating process.

Further, a method for producing asialoerythropoietin according to the present invention is a method for producing asialoerythropoietin, including a sialic acid removing process of removing a sialic acid bonded to erythropoietin by the method for removing a sialic acid according to the present invention.

### Effects of the Invention

The inventor of the present invention conducted keen studies of a method for removing a sialic acid bonded to erythropoietin, and found that it is possible to remove the sialic acid merely by acidifying and heating the erythropoietin, without harming its biological activity, thus arriving at the present invention.

Since the method for removing a sialic acid and the method for producing asialoerythropoietin according to the present invention can be carried out simply at a low cost, it is possible to produce asialoerythropoietin simply and inexpensively. Also, since the asialoerythropoietin produced by the production process according to the present invention performs a biological function such as stimulating cell proliferation that is comparable with the function of erythropoietin, it can be utilized widely in the field of medicine such as therapy or production of pharmaceutical compositions.

### Brief Description of Drawings

[FIG. 1] FIG. 1 is a photograph showing gels that were subjected to SDS-PAGE for asialo EPO and native EPO (Example 1).
[FIG. 2] FIG. 2 is a photograph showing gels that were subjected to IEF for asialo EPO and native EPO (Example 1).
[FIG. 3] FIG. 3 is a graph comparing a function of stimulating cell proliferation of native EPO and that of asialo EPO (Example 1).

### Description of the Invention

In the method for removing a sialic acid according to the present invention, it is preferable that the acidified solution is at pH 4.0 or lower. Also, it is preferable that the heating is carried out for raising a temperature of the solution to at least 60°C.

In the method for removing a sialic acid according to the present invention, it is preferable that a treatment period of the acidifying and heating process is at least 15 minutes.

It is preferable that the method for removing a sialic acid according to the present invention further includes a neutralizing and cooling process of neutralizing and cooling the solution containing the erythropoietin after the acidifying and heating process.

It is preferable that the neutralizing is carried out for making a pH of the solution range from pH 5.0 to 10.0. Also, it is preferable that the cooling is carried out for making a temperature of the solution range from 0°C to 50°C.

In the present invention, "asialoerythropoietin (in the following, also referred to as asialo EPO)" refers to erythropoietin (in the following, also referred to as EPO) obtained by removing a sialic acid bonded to an EPO molecule.

Usually, both of EPO produced from genetic recombinant animal cells and EPO derived from urine are obtained as EPO compositions containing various EPOs with different sugar chain structures. Similarly, the number of sialic acids bonded to an EPO molecule in the EPO composition also varies depending on individual EPO molecules and usually is 11 to 15 for a single EPO molecule. In the present invention, the asialo EPO is obtained by removing at least one of these sialic acids. There is no particular limitation on the number of the sialic acids to be removed from the EPO by the above-mentioned sialic acid removal. For example, all the sialic acids may be removed, or 1, 2, 3, 4, 5, 6, 7, 8, 9, 10, 11, 12, 13 or 14 sialic acids may be removed. For the asialo EPO in the present invention, the number of the sialic acids bonded to the EPO molecule preferably is equal to or smaller than 10, more preferably is equal to or smaller than 5 and particularly preferably is equal to or smaller than 2.

Incidentally, the number of sialic acids bonded to the EPO and the asialo EPO can be expressed by an average number per EPO molecule contained in the EPO composition, and this average number of the sialic acids per molecule can be measured by a method conventionally known to a person having an ordinary skill in the art (for example, JP 8(1996)-151398 A, EP 0428267). For instance, EPO can be hydrolyzed using 0.35 M sulfuric acid at 80°C for 30 minutes so as to cut off all the sialic acids from the EPO, followed by quantification of each of the proteins and the sialic acids of the EPO, thereby calculating the number of moles of the sialic acid per mole of the EPO.

The EPO used for the present invention can be any EPO and preferably is highly purified EPO. More specifically, EPO having substantially the same biological activity as mammal EPO, in particular, human EPO is preferable, for example.

The above-noted method for producing EPO is not particularly limited and can be, for example, a process of purifying a human-derived extract so as to obtain native human EPO (for example, see JP 1(1989)-38800 B (corresponding to WO 86/04068)) or a process of using human EPO, etc. that is expressed in cells such as E. coli, yeast, chinese hamster ovary cell (CHO cell), C127 cell, COS cell, myeloma cell, BHK cell and insect cell by genetic engineering including a genetic recombination, extracted, isolated and purified by various methods. Among them, the EPO used in the present invention preferably is EPO that is produced by genetic engineering and preferably is EPO that is produced using mammal cell, in particular, CHO cell (for example, see JP 1(1989)-44317 A (corresponding to WO 86/03520), and Kenneth Jacobs et al., Nature, 313 806-810 (1985)).

The EPO produced by genetic engineering includes EPO that has the same amino acid sequence as native EPO or that has an amino acid sequence obtained by deletion, substitution, addition, etc. of one amino acid or a plurality of amino acids in the above-noted amino acid sequence, and that has a biological activity similar to the native EPO. The deletion, substitution, addition, etc. of amino acids can be conducted by a method conventionally known to any one of ordinary skill in the art. For example, by using site-directed mutagenesis (Gotoh, T. et al. (1995) Gene 152, 271-275; Zoller, M.J. and Smith, M. (1983) Methods Enzymol. 100, 468-500; Kramer, W. et al. (1984) Nucleic Acids Res. 12, 9441-9456; Kramer, W. and Fritz, H.J. (1987) Methods Enzymol. 154, 350-367; Kunkel, T.A. (1985) Proc. Natl. Acad. Sci. USA. 82, 488-492; Kunkel (1988) Methods Enzymol. 85, 2763-2766), etc., mutation is introduced in the amino acid sequence of the EPO suitably, thereby preparing polypeptides functionally similar to the EPO. Also, the amino acid mutation also can occur in the realm of nature. In general, it is preferable that an amino acid residue to be substituted is substituted by another amino acid in which the property of an amino acid side chain is retained. For example, such a property of the amino acid side chain can be a hydrophobic amino acid (A, I, L, M, F, P, W, Y, V; which are single character notations of amino acids and used similarly below), a hydrophilic amino acid (R, D, N, C, E, Q, G, H, K, S, T), an amino acid having an aliphatic side chain (G, A, V, L, I, P), an amino acid having a hydroxyl group-containing side chain (S, T, Y), an amino acid having a sulfur atom-containing side chain (C, M), an amino acid having a carboxylic acid and an amide-containing side chain (D, N, E, Q), an amino acid having a base-containing side chain (R, K, H), an amino acid having an aromatic side chain (H, F, Y, W), etc. It already has been known that polypeptides having an amino acid sequence that is modified by deletion, addition, substitution, etc. of one amino acid residue or a plurality of amino acid residues for a certain amino acid sequence can maintain its biological activity (Mark, D.F. et al., Proc. Natl. Acad. Sci. USA (1984) 81, 5662-5666; Zoller, M.J. & Smith, M. Nucleic Acids Research (1982) 10, 6487-6500; Wang, A. et al., Science 224, 1431-1433; Dalbadie-McFarland, G. et al., Proc. Natl. Acad. Sci. USA (1982) 79, 6409-6413).

The EPO used in the present invention may be a fusion protein of EPO and another protein. A fusion polypeptide can be produced by, for example, joining a DNA encoding the EPO and a DNA encoding the other protein such that their frames coincide with each other, inserting them in an expression vector and allowing them to be expressed in a host. The above-noted other protein is not particularly limited. Also, the EPO used in the present invention may be chemically-modified EPO or EPO whose sugar chain is modified and altered. Examples of the chemically-modified EPO include EPO obtained by bonding inorganic or organic compounds such as polyethylene glycol or vitamin B12. Furthermore, the EPO used in the present invention also can be EPO in which an amino acid in the molecule is modified. The amino acid in the EPO molecule can be modified by, for example, carbamoylation, biotinylation, amidination, acetylation, guanidination, etc.

Now, the following is a description of the method for removing a sialic acid according to the present invention.

The method for removing a sialic acid according to the present invention includes an acidifying and heating process of treating an EPO-containing solution under an acid heated condition, thereby removing a sialic acid bonded to an EPO molecule.

The concentration of EPO of the above-noted EPO-containing solution is not particularly limited and, for example, 0.01 mg/l to 100.0 mg/l, preferably 0.05 mg/l to 50.0 mg/l and more preferably 1.0 mg/l to 10.0 mg/l.

The above-noted acidified solution is, for example, at pH 4.0 or lower, preferably at pH 0.1 to pH 4.0, more preferably at pH 0.5 to pH 3.0 and further more preferably at pH 1.0 to pH 2.5.

There is no particular limitation on how to acidify the above-noted EPO-containing solution into the above range. A method known to a person having an ordinary skill in the art can be employed. For example, an acid solution such as a hydrochloric acid solution, a phosphoric acid solution or an acetic acid solution can be added to the EPO-containing solution.

The above-mentioned heating is for treating the EPO-containing solution in order to remove a sialic acid and carried out at a temperature of, for example, at least 60°C, preferably 60°C to 100°C, more preferably 70°C to 90°C and further preferably 75°C to 85°C.

In the above-mentioned acidifying and heating process, there is no particular limitation on the order of a process of acidifying the EPO-containing solution and a process of heating the same. The EPO-containing solution may be acidified and then heated, the EPO-containing solution may be heated and then acidified, or the acidifying process and the heating process may be carried out at the same time.
Among them, in the present invention, it is preferable to carry out the process of acidifying the EPO-containing solution and then the process of heating the same.

In the method for removing a sialic acid according to the present invention, a treatment period of the above-described acidifying and heating process for removing a sialic acid bonded to an EPO molecule is not particularly limited and, for example, at least 15 minutes, preferably at least 30 minutes, more preferably at least 45 minutes and further more preferably at least 60 minutes. The upper limit of the treatment period is not particularly limited and, for example, 24 hours, preferably 360 minutes and further preferably 120 minutes.

It is preferable that the method for removing a sialic acid according to the present invention further includes a neutralizing and cooling process of neutralizing and cooling the EPO-containing solution after the above-described acidifying and heating process.

The above-mentioned neutralizing is carried out for making the pH of the solution range from, for example, pH 5.0 to pH 10.0. The pH achieved by the neutralizing is preferably pH 6.0 to pH 9.0 and more preferably pH 6.5 to pH 7.5.

There is no particular limitation on how to neutralize the above-noted EPO-containing solution. A method known to a person having an ordinary skill in the art can be employed, for example, an alkaline solution such as a sodium hydroxide solution can be added to the solution after the process of removing a sialic acid.

The above-mentioned cooling is carried out for making a temperature of the solution range from, for example, 0°C to 50°C, preferably 0°C to 40°C and further preferably 0°C to 30°C.

In the above-mentioned neutralizing and cooling process, there is no particular limitation on the order of a process of neutralizing the EPO-containing solution and a process of cooling the same. The EPO-containing solution may be neutralized and then cooled down, it may be cooled down and then neutralized, or the neutralizing process and the cooling process may be carried out at the same time. Among them, in the present invention, it is preferable to carry out the neutralizing process and then the cooling process.

Now, the method for producing asialoerythropoietin according to the present invention will be described.

The method for producing asialo EPO according to the present invention includes a sialic acid removing process of removing a sialic acid bonded to erythropoietin by the method for removing a sialic acid according to the present invention.

A treatment condition of acidifying and heating the EPO-containing solution, a method therefor, an order thereof and a treatment period in the method for removing a sialic acid are as described above.

Similarly, a treatment condition of neutralizing and cooling the EPO-containing solution, a method therefor and an order thereof in the method for removing a sialic acid are as described above.

Asialo EPO in an asialo EPO-containing solution obtained by the above-described method for removing a sialic acid can be purified suitably by, for example, a method known to a person having an ordinary skill in the art, thus obtaining a highly purified asialo EPO.

Since the asialo EPO produced by the method for producing asialo EPO according to the present invention has a biological activity equivalent to EPO, it can be used for a pharmaceutical composition, a reagent, etc., for example. In the present invention, the biological activity equivalent to EPO is an activity that EPO has and can be, for example, an activity of stimulating cell proliferation, promotion of regeneration of cells and tissues, an activity of protecting cells and tissues, etc., though there is no particular limitation.

When the asialo EPO is used for a pharmaceutical composition, it also may be possible to add a suspending agent, a solubilizing agent, a stabilizing agent, an isotonizing agent, a preservative, an adsorption preventing agent, a surfactant, a diluent, an excipient, a pH controlling agent, a soothing agent, a buffer, a sulfur-containing reducing agent, an antioxidant, etc., as necessary.

The above-noted suspending agent is not particularly limited and can be, for example, methyl cellulose, polysorbate 80, hydroxyethyl cellulose, gum Arabic, powdered tragacanth, sodium carboxymethylcellulose, polyoxyethylene sorbitan monolaurate or the like.

The above-noted solubilizing agent is not particularly limited and can be, for example, polyoxyethylene hydrogenated castor oil, polysorbate 80, nicotinic acid amide, polyoxyethylene sorbitan monolaurate, macrogol, castor oil fatty acid ethyl ester or the like.

The above-noted stabilizing agent is not particularly limited and can be, for example, dextran 40, methyl cellulose, gelatin, sodium sulfite, sodium metasulfite or the like. It also is possible to add a certain kind of an amino acid as the above-noted stabilizing agent (for example, see JP 10(1998)-182481 A). Examples of the amino acid to be added as the stabilizing agent include a free amino acid and salts thereof such as a sodium salt, a potassium salt and a hydrochloride salt. It is possible to add one kind of the above-noted amino acid or two or more kinds thereof in combination. The kinds are not particularly limited, and examples of a preferable amino acid include leucine, tryptophan, serine, glutamic acid, arginine, histidine and lysine.

The above-noted isotonizing agent is not particularly limited and can be, for example, D-mannitol, sorbitol or the like.

The above-noted preservative is not particularly limited and can be, for example, methyl parahydroxybenzoate, ethyl parahydroxybenzoate, sorbic acid, phenol, cresol, chlorocresol or the like.

The above-noted adsorption preventing agent is not particularly limited and can be, for example, human serum albumin, lecithin, dextran, ethylene oxide-propylene oxide copolymer, hydroxypropylcellulose, methyl cellulose, polyoxyethylene hydrogenated castor oil, polyethylene glycol or the like.

The above-noted surfactant is not particularly limited and typically can be nonionic surfactant, anionic surfactant, a natural surfactant or the like. The above-mentioned nonionic surfactant can be, for example, sorbitan fatty acid ester such as sorbitan monocaprylate, sorbitan monolaurate or sorbitan monopalmitate; glycerin fatty acid ester such as glycerin monocaprylate, glycerin monomyristate or glycerin monostearate; polyglycerin fatty acid ester such as decaglycermonostearate, decaglycerdistearate or decaglycerylmonolinoleate; polyoxyethylene sorbitan fatty acid ester such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate or polyoxyethylene sorbitan tristearate; polyoxyethylene sorbitol fatty acid ester such as polyoxyethylene sorbitol tetrastearate or polyoxyethylene sorbitol tetraoleate; polyoxyethylene glycerin fatty acid ester such as polyoxyethylene glycerin monostearate; polyethylene glycol fatty acid ester such as polyethylene glycol distearate; polyoxyethylene alkyl ether such as polyoxyethylene lauryl ether; polyoxyethylene polyoxypropylene alkyl ether such as polyoxyethylene polyoxypropylene glycol, polyoxyethylene polyoxypropylene propyl ether or polyoxyethylene polyoxypropylene cetyl ether; polyoxyethylene alkyl phenyl ether such as polyoxyethylene nonyl phenyl ether; polyoxyethylene hydrogenated castor oil such as polyoxyethylene castor oil or polyoxyethylene hydrogenated castor oil; polyoxyethylene bees wax derivatives such as polyoxyethylene sorbitol bees wax; polyoxyethylene lanolin derivatives such as polyoxyethylene lanolin; polyoxyethylene fatty acid amide such as polyoxyethylene stearic acid amide; or these having HLB of 6 to 18. The above-mentioned anionic surfactant can be, for example, alkyl sulfate having C10 to C18 alkyl groups such as sodium cetyl sulfate, sodium lauryl sulfate or sodium oleyl sulfate; polyoxyethylene alkyl ether sulfate having an average number of added moles of ethylene oxide of 2 to 4 and having C10 to C18 alkyl groups such as sodium polyoxyethylene lauryl sulfate; or alkyl sulfosuccinate having C8 to C18 alkyl groups such as sodium lauryl sulfosuccinate. The above-mentioned natural surfactant can be, for example, lecithin, glycerophospholipid; sphingophospholipid such as sphingomyelin; or sucrose fatty acid ester having C12 to C18 fatty acids. These surfactants may be added alone or in combination of two or more kinds. Among them, polyoxyethylene sorbitan fatty acid ester such as polysorbate 20, 40, 60 or 80 are preferable, and polysorbate 20 and polysorbate 80 are particularly preferable. Further, polyoxyethylene polypropylene glycol represented by poloxamer (Pluronic F-68 (registered trademark), etc.) also is preferable.

The above-noted sulfur-containing reducing agent is not particularly limited and can be, for example, those having a sulfhydryl group such as N-acetylcysteine, N-acetylhomocysteine, thioctic acid, thiodiglycol, thioethanolamine, thioglycerol, thiosorbitol, thioglycolic acid and a salt thereof, sodium thiosulfate, glutathione, thioalkanoic acid having 1 to 7 carbon atoms.

The above-noted antioxidant is not particularly limited and can be, for example, erythorbic acid, dibutyl hydroxytoluene, butylhydroxyanisol, α-tocopherol, tocopherol acetate, L-ascorbic acid and a salt thereof, L-ascorbyl palmitate, L-ascorbyl stearate, sodium hydrogensulfite, sodium sulfite, triamyl gallate, propyl gallate, or a chelating agent such as disodium ethylenediaminetetraacetate (EDTA), sodium pyrophosphate or sodium metaphosphate.

Furthermore, it also may be possible to add a component that is added usually, for example, an inorganic salt such as sodium chloride, potassium chloride, calcium chloride, sodium phosphate, potassium phosphate or sodium hydrogencarbonate or an organic salt such as sodium citrate, potassium citrate or sodium acetate.

When a pharmaceutical composition containing the above-described asialo EPO is administered, the amount of administration is such that the asialo EPO is, for example, 0.001 µg/kg/day to 1000 µg/kg/day, preferably 0.01 µg/kg/day to 100 µg/kg/day and further preferably 0.1 µg/kg/day to 30 µg/kg/day. However, the amount of administration is not limited to this and can be determined by a medical doctor considering the age, weight, gender, symptom of a patient and the route of administration. Therefore, the present invention may include as the other aspects a method for producing an asialo EPO-containing pharmaceutical composition including a process of producing an asialo EPO by the method for producing an asialo EPO according to the present invention, the above-described pharmaceutical composition produced thereby, and a therapeutic method including administering the above-described pharmaceutical composition, for example, a method of a therapy for anemia and a management before and after an operation.

In the following, the present invention will be described further by way of an example.

### Example 1

### (Production of asialo EPO)

First, 190 µl of a native EPO-containing solution (1300 µg/ml) was prepared. After 10 µl of 1.0 N HCl was added to the above solution, the solution was heated to 80°C and incubated for 60 minutes, thereby removing a sialic acid bonded to the EPO. Subsequently, 2.0 µl of 5.0 N NaOH was added for neutralization and cooled down to room temperature, thus obtaining an asialo EPO-containing solution. The pH of the solution after the above-described HCl addition was 1.0, and the pH of the solution after the above-described NaOH addition was 7.0. Incidentally, the above-noted native EPO was obtained by culturing CHO cells in which EPO genes had been introduced.

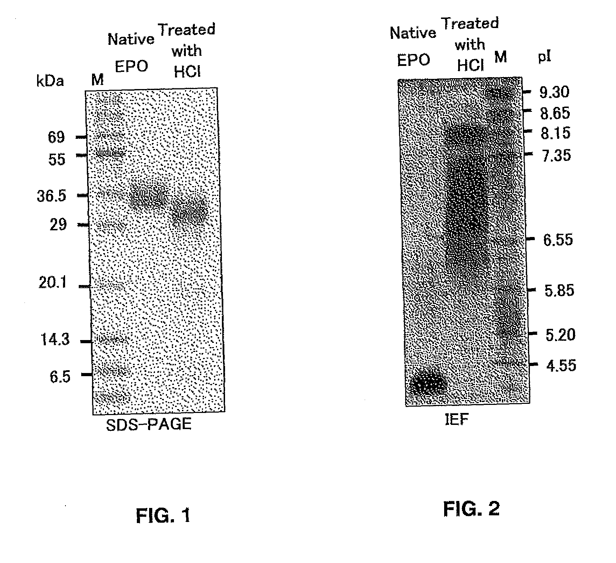

The molecular weight and the isoelectric point of each of the native EPO and the asialo EPO after sialic acid removal were examined respectively by SDS polyacrylamide gel electrophoresis (SDS-PAGE) and isoelectric focusing (IEF). FIGs. 1 and 2 show their results, respectively. FIG. 1 is a photograph showing gels in which proteins were dyed after the SDS-PAGE. As shown in this figure, the molecular weight of the asialo EPO was smaller than that of the native EPO before sialic acid removal. Also, FIG. 2 is a photograph showing gels in which proteins were dyed after the IEF. As shown in this figure, the isoelectric point of the native EPO before sialic acid removal was lower than 4.55, while that of the asialo EPO after sialic acid removal was equal to or higher than 7.35. This change corresponds to the removal of a sialic acid from an EPO molecule. Further, the average number of sialic acids for one produced asialo EPO molecule was 0.

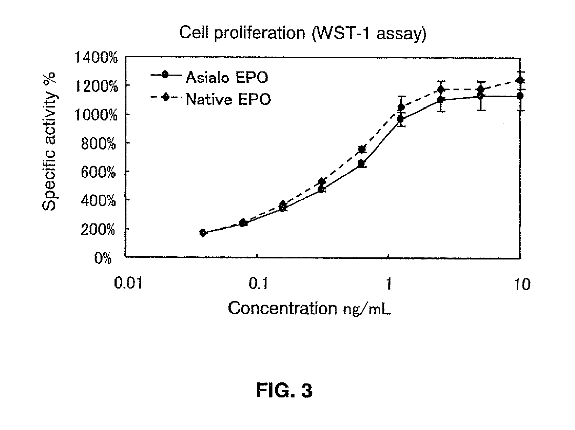

In order to compare the biological activity of thus produced asialo EPO with the biological activity of the native EPO, examination was conducted using an activity of stimulating the proliferation of an EPO-dependent cell line as an index. The AS-E2 cells used for this test were an EPO-receptor expressing cell line that was established from a bone marrow cell of a human leukemia patient and had a characteristic of not being able to exist in the absence of EPO. The asialo EPO and the native EPO described above were added respectively, and the influence thereof on the cell proliferation was determined by WST-1 assay. FIG. 3 shows the result thereof. As shown in this figure, both of them had similar functions of stimulating the cell proliferation.

### Industrial Applicability

Since the method for removing a sialic acid and the method for producing asialoerythropoietin according to the present invention can be carried out simply at a low cost as described above, they can be utilized for producing pharmaceutical compositions, for example, and are useful widely in the field of medicine.

## Claims

1. A method for removing a sialic acid bonded to erythropoietin, comprising an acidifying and heating process of acidifying and heating a solution containing the erythropoietin.

2. The method for removing a sialic acid according to claim 1, further comprising a neutralizing and cooling process of neutralizing and cooling the solution containing the erythropoietin after the acidifying and heating process.

3. The method for removing a sialic acid according to claim 1 or 2, wherein the acidified solution is at pH 4.0 or lower.

4. The method for removing a sialic acid according to any one of claims 1 to 3, wherein the heating is carried out for raising a temperature of the solution to at least 60°C.

5. The method for removing a sialic acid according to any one of claims 1 to 4, wherein a treatment period of the acidifying and heating process is at least 15 minutes.

6. The method for removing a sialic acid according to any one of claims 2 to 5, wherein the neutralizing is carried out for making a pH of the solution range from pH 5.0 to 10.0.

7. The method for removing a sialic acid according to any one of claims 2 to 6, wherein the cooling is carried out for making a temperature of the solution range from 0°C to 50°C.

8. A method for producing asialoerythropoietin, comprising a sialic acid removing process of removing a sialic acid bonded to erythropoietin by the method for removing a sialic acid according to any one of claims 1 to 7.
